(19) Europäisches Patentamt
European Patent Office
Office européen des brevets

(11) **EP 1 858 828 B1**

(12) **EUROPÄISCHE PATENTSCHRIFT**

(45) Veröffentlichungstag und Bekanntmachung des Hinweises auf die Patenterteilung:
**02.03.2016 Patentblatt 2016/09**

(21) Anmeldenummer: **06723261.1**

(22) Anmeldetag: **07.03.2006**

(51) Int Cl.:
***C07C 1/24*** *(2006.01)*

(86) Internationale Anmeldenummer:
**PCT/EP2006/002062**

(87) Internationale Veröffentlichungsnummer:
**WO 2006/097222 (21.09.2006 Gazette 2006/38)**

(54) **VERFAHREN ZUR DEHYDRATISIERUNG VON FETTALKOHOLEN**

METHOD FOR DEHYDRATING FATTY ALCOHOLS

PROCEDE POUR LA DESHYDRATATION D'ALCOOLS GRAS

(84) Benannte Vertragsstaaten:
**DE ES FR GB IT**

(30) Priorität: **16.03.2005 DE 102005012049**

(43) Veröffentlichungstag der Anmeldung:
**28.11.2007 Patentblatt 2007/48**

(73) Patentinhaber: **Cognis IP Management GmbH
40589 Düsseldorf (DE)**

(72) Erfinder: **DIERKER, Markus
40597 Düsseldorf (DE)**

(56) Entgegenhaltungen:
**EP-A- 1 333 017          DE-A1- 3 911 004
DE-A1- 19 511 668      US-A- 4 165 343
US-A- 4 873 392          US-A- 5 659 102**

Bemerkungen:
Die Akte enthält technische Angaben, die nach dem Eingang der Anmeldung eingereicht wurden und die nicht in dieser Patentschrift enthalten sind.

EP 1 858 828 B1

## Beschreibung

### Gebiet der Erfindung

Die Erfindung betrifft ein Verfahren zur Herstellung von Kohlenwasserstoffen durch Dehydratisierung von primären Fettalkoholen.

### Stand der Technik

Der Einsatz von Kohlenwasserstoffen in kosmetischen Zusammensetzungen ist seit langem bekannt. So werden beispielsweise Mineralöle und Paraffinöle als inerte Ölkörper eingesetzt. Diese haben den Nachteil, dass sie aus sensorischer Sicht ein "schweres" Hautgefühl hinterlassen und schlecht spreiten.

Zahlreiche Verfahren zur Herstellung von Kohlenwasserstoffen gehen dabei von kurzkettigen Olefinen aus, die oligomerisiert werden. Methylverzweigte Paraffine und deren Herstellung aus Guerbetalkoholen in Gegenwart von Alkylslufonsäure-Katalysatoren sind beispielsweise Gegenstand der DE 3911004**x.** Bekannt ist auch der Einsatz von Produktmischungen verschiedenster Kohlenwasserstoffe mit verbesserter Spreitfähigkeit, wie sie beispielsweise gemäß der in DE 103 17 781 oder DE 103 24 508 beschriebenen Verfahren erhältlich sind. Derartige Gemische sind nur schwer charakterisierbar und enthalten eine Vielzahl verschiedener Einzelkomponenten mit höheren Oligomeren. Verschiedenartige Verfahren zur Dehydratisierung von Alkoholen sind seit langem bekannt und Lehrbuchwissen (vgl. Organikum). Allerdings funktionieren viele dieser Verfahren oft nur bei sehr hohen Temperaturen (z.B. GB 2181070) oder mit vergleichsweise ungewöhnlichen und teuren Katalysatoren, wie Niob- oder Tantalsäure (WO 97/03932). DE 195 11 668 A1 beschreibt ein Verfahren zur Herstellung von Dialkylethern, bei dem man Halogenalkansulfonsuren als katalysator einsetzt.

Aufgabe der vorliegenden Erfindung war es daher, ein Verfahren zur Herstellung von Kohlenwasserstoffen bereitzustellen, welches sich unter schonenden Bedingungen durchführen lässt und bei dem weniger Nebenreaktionen, wie Pyrolyse oder Etherbildung, stattfinden. Überraschenderweise wurde gefunden, dass dies durch den Einsatz von Trifuormethansulfonsäure als Katalysator möglich ist.

### Beschreibung der Erfindung

Gegenstand der Erfindung ist ein Verfahren zur Herstellung von Kohlenwasserstoffen durch Dehydratisierung primärer Alkohole, wobei als Dehydratisierungs-Katalysator 0,5-3 gew.% Trifluormethansulfonsäure eingesetzt wird. Als primäre Alkohole geeignet sind beispielsweise Hexanol, Heptanol, Octanol, Nonanol, Decanol, Undecanol, Undecenol, Dodecanol, Tridecanol, Tetradecanol, Pentadecanol, Hexadecanol, Heptadecanol, Octadecanol, Octadecenol, Nonadecanol, Eicosanol, Eicosenol, Heneicosanol, Docosanol und Docosenol. Auch technische Gemische von primären Alkoholen, wie sie mittels üblicher fettchemischer Methoden aus natürlichen Fetten und Ölen erhältlich sind, lassen sich erfindungsgemäß einsetzen. Hierzu zählen Capron-, Önanth-, Capryl-, Pelargon-, Caprin-, Lauryl-, Myristyl-, Cetyl-, Stearyl-, Arachidyl-, Behenyl-, Oleyl-, Elaidyl-, Linoleyl-, Gadoleyl-, Arachidon-, Eruca- und Brassidylalkohol erhalten.

Vorzugsweise werden als primäre Alkohole lineare oder verzweigte, gesättigte oder ungesättigte C6-C30-Alkohole eingesetzt. Vorzugsweise werden primäre C6-C18-Alkohole eingesetzt, vorzugsweise C10-C14-Alkohole. Die Reaktion funktioniert für letztere besonders gut in hohen Ausbeuten. In einer bevorzugten Ausführungsvariante des Verfahrens sind die Alkohole ausgewählt aus den verzweigten C7-C13-Alkoholen. Zu letzteren zählen Guerbetalkohole wie 2-Ethylhexanol und insbesondere die von der Firma Exxon vermarkteten Exxal® - Alkohole, wie beispielsweise Isoheptanol (Exxal® 7), Isooctanol (Exxal® 8n), Isononanol (Exxal® 9), Isodecanol (Exxal® 10), Isoundecanol (Exxal® 11), Isotridecanol (Exxal® 13). Zu den erfindungsgemäß einsetzbaren Guerbetalkoholen zählt neben 2-Ethylhexanol, 2-Propylheptanol, 2-Butyloctanol und 2-Hexyldecanol.

Das erfindungsgemäße Verfahren zur Dehydratisierung wird üblicherweise bei Temperaturen im Bereich von 190 - 260°C, vorzugsweise im Bereich von 200 - 250°C durchgeführt und ganz besonders bevorzugt im Bereich von 220 - 250°C. In einer weiteren bevorzugten Variante des erfindungsgemäßen Verfahrens wird die Dehydratisierung unter Unterdruck durchgeführt. Dadurch kann die Reaktionstemperatur weiter abgesenkt werden, so dass weniger Nebenreaktionen auftreten. Insbesondere bei längerkettigen Alkoholen ist dies vorteilhaft. Erfindungsgemäß vorteilhaft kann es auch sein, die Reaktion unter Schutzgas, wie beispielsweise Stickstoff oder Argon, durchzuführen.

Erfindungsgemäß ist es, für die Dehydratisierung 0,5 - 3 Gew.-% des Katalysators bezogen auf die Menge des Alkohols einzusetzen.

### Gewerbliche Anwendbarkeit

Die mit dem erfindungsgemäßen Verfahren hergestellten Kohlenwasserstoffe können in einer Vielzahl von Applikationen eingesetzt werden, z.B. in kosmetischen Zusammensetzungen, in Möbelpolituren, in Textilbehandlungsmitteln, etc. Die längerkettigen C10-C30-Kohlenwasserstoffe, insbesondere die C10-C20-Kohlenwasserstoffe, eignen sich besonders gut zur Herstellung kosmetischer Zusammensetzungen zur Körperpflege und -reinigung und lassen sich in Cremes, Lotionen, sprühbaren Emulsionen, Produkten zur Eliminierung des Körpergeruchs, Schaum- und Duschbäder, Haarshampoos und Pflegespülungen einsetzen.

**Beispiele**

Allgemeine Reaktionsvorschrift

Der Alkohol wird mit dem Katalysator (0,5-3%) zusammen in einer Destillationsapparatur vorgelegt und mehrere Stunden auf 240°C erwärmt. Abdestilliertes Edukt oder Produkt kann über einen Wasserabscheider wieder zurück in das Reaktionsgefäß geführt werden. Die organische Phase des erhaltenen Destillats wird über Natriumsulfat getrocknet und das Produkt durch Filtration isoliert. Die Ausbeute beträgt üblicherweise > 80% des gewünschten Olefins.

Beispiel 1: Dodecen

600,0 g Lorol® C12 (Dodecanol, von Cognis Deutschland GmbH & Co. KG) und 18,0 g Trifluormethansulfonsäure (25 Gew.-% in Wasser) werden in einer Destillationsapparatur vorgelegt und 4h auf 240°C erwärmt. Die organische Phase des erhaltenen Destillats (476,0 g) wird über Natriumsulfat getrocknet und das Produkt durch Filtration isoliert. Das Produkt enthält 93,3 % Dodecen, 4,6% Dodecanol und 1,1% Didodecylether (GC-Analyse).

Beispiel 2: Isododecen

300,0 g Lial® 123 (Isododecanol; von Sasol) und 18,0 g Trifluormethansulfonsäure (50 Gew.% in Wasser) werden in einer Destillationsapparatur vorgelegt und 1,5h auf 240°C erwärmt. Die organische Phase des erhaltenen Destillats (171,9 g) wird über Natriumsulfat getrocknet und das Produkt durch Filtration isoliert. Das Produkt enthält anschließend 91,5 % Isododecen, 7,5 % Lial® 123 und 1,0% Didodecylether (GC-Analyse).

Beispiel 3: Isotridecen

1200,0 g Exxal® 13 (Isotridecanol; von Exxon Mobile) und 12,0 g Trifluormethansulfonsäure (25 % Gew.-% in Wasser) werden in einer Destillationsapparatur vorgelegt und 5h auf 240°C erwärmt. Die organische Phase des erhaltenen Destillats (994,6 g) wird über Natriumsulfat getrocknet und das Produkt durch Filtration isoliert. Das Produkt enthält 94,0 % Isotridecen und 6,0 % Isotridecanol (GC-Analyse).

$$JZ \text{ (Kaufmann)} = 194, JZ \text{ (Wijs)} = 241, OHZ = 22,5$$

Beispiel 4: Tetradecen

600,0 g Lorol® C14 (Tetradecanol) und 12,0 g Trifluormethansulfonsäure (25 Gew.-% in Wasser) werden in einer Destillationsapparatur vorgelegt und 5h auf 240 °C erwärmt. Die organische Phase des erhaltenen Destillats (308,0 g) wird über Natriumsulfat getrocknet und das Produkt durch Filtration isoliert. Das Produkt enthält anschließend 84,3 % Tetradecen, 10,8 % Tetradecanol und 4,8 % Ditetradecylether (GC-Analyse).

**Patentansprüche**

1. Verfahren zur Herstellung von Kohlenwasserstoffen durch Dehydratisierung primärer Alkohole, wobei als Dehydratisierungs-Katalysator Trifluormethansülfonsäure eingesetzt wird, **dadurch gekennzeichnet, dass** für die die Dehydratisierung 0,5 - 3 Gew.-% des Katalysators eingesetzt werden.

2. Verfahren gemäß Anspruch 1, **dadurch gekennzeichnet, dass** als primäre Alkohole lineare oder verzweigte, gesättigte oder ungesättigte C6-C30-Alkohole eingesetzt werden.

3. Verfahren gemäß wenigstens einem der Ansprüche 1 oder 2, **dadurch gekennzeichnet, dass** die Alkohole ausgewählt sind aus den verzweigten C7-C13-Alkoholen.

**4.** Verfahren gemäß wenigstens einem der Ansprüche 1 bis 3, **dadurch gekennzeichnet, dass** die Dehydratisierung bei einer Temperatur im Bereich von 200 - 250 °C durchgeführt wird.

**5.** Verfahren gemäß wenigstens einem der Ansprüche 1 bis 4, **dadurch gekennzeichnet, dass** die Dehydratisierung unter Unterdruck durchgeführt wird.

**Claims**

**1.** A process for preparing hydrocarbons by dehydrating primary alcohols, using trifluoromethanesulfonic acid as dehydration catalyst, wherein 0.5%-3% by weight of the catalyst is used for the dehydration.

**2.** The process as claimed in claim 1, wherein the primary alcohols used are linear or branched, saturated or unsaturated C6-C30 alcohols.

**3.** The process as claimed in at least one of claims 1 and 2, wherein the alcohols are selected from the branched C7-C13 alcohols.

**4.** The process as claimed in at least one of claims 1 to 3, wherein the dehydration is conducted at a temperature in the range of 200-250°C.

**5.** The process as claimed in at least one of claims 1 to 4, wherein the dehydration is conducted under reduced pressure.

**Revendications**

**1.** Procédé de fabrication d'hydrocarbures par déshydratation d'alcools primaires, de l'acide trifluorométhanesulfonique étant utilisé en tant que catalyseur de déshydratation, **caractérisé en ce que** 0,5 à 3 % en poids du catalyseur est utilisé pour la déshydratation.

**2.** Procédé selon la revendication 1, **caractérisé en ce que** des alcools en C6-C30 linéaires ou ramifiés, saturés ou insaturés, sont utilisés en tant qu'alcools primaires.

**3.** Procédé selon au moins l'une quelconque des revendications 1 ou 2, **caractérisé en ce que** les alcools sont choisis parmi les alcools en C7-C13 ramifiés.

**4.** Procédé selon au moins l'une quelconque des revendications 1 à 3, **caractérisé en ce que** la déshydratation est réalisée à une température dans la plage allant de 200 à 250 °C.

**5.** Procédé selon au moins l'une quelconque des revendications 1 à 4, **caractérisé en ce que** la déshydratation est réalisée à une sous-pression.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3911004
- DE 10317781
- DE 10324508
- GB 2181070 A
- WO 9703932 A
- DE 19511668 A1